# EUROPÄISCHE PATENTSCHRIFT

(11) **EP 0 741 998 B1**
(45) Veröffentlichungstag und Bekanntmachung des Hinweises auf die Patenterteilung: **17.03.1999**
(21) Anmeldenummer: 96106337.7
(22) Anmeldetag: 23.04.1996
(51) Int. Cl.: A61F 13/06

(54) **Selbstklebende Fertigbandage für die Patella**
Adhesive bandage for the patella
Bandage adhésif pour la rotule

(30) Priorität: 11.05.1995 DE 19517209
(43) Veröffentlichungstag der Anmeldung: 13.11.1996
(73) Patentinhaber: Beiersdorf Aktiengesellschaft, 20245 Hamburg (DE)
(72) Erfinder: Staudinger, Peter, 25436 Tornesch (DE)

(56) Entgegenhaltungen:
- EP-A- 0 519 135
- WO-A-93/00788
- FR-A- 2 307 518
- GB-A- 2 242 818
- US-A- 2 983 272
- US-A- 4 748 975
- US-A- 5 139 476
- US-A- 5 370 606

## Beschreibung

Die Erfindung betrifft eine einseitig selbstklebend beschichtete Fertigbandage zur Stabilisierung und Führung der Patella in ihrem natürlichen Gleitlager.

Die funktionelle Verbandtechnik, das sogenannte Taping, ist eine Behandlungsmethode zur Prophylaxe und Therapie von Verletzungen, Krankheiten und Veränderungen am Bewegungsapparat. Taping hat zum Ziel, die Kapsel-Band-Strukturen gezielt nachzubilden und dadurch eine selektive Unterstützung und Stabilisierung zu erreichen.

Der eigentliche Tapeverband wird dabei streifenweise aus vorzugsweise unelastischen selbstklebenden Bändern, sogenannten Zügeln, oder in Verbindung mit kurtzugelastischen selbstklebenden Bändern angelegt. Er schützt, stützt und entlastet gefährdete, geschädigte oder gestörte Anteile einer Funktionseinheit. Er erlaubt die funktionelle Belastung im schmerzfreien Bewegungsraum, verhindert aber extreme oder schmerzhafte Bewegungen.

Das Anlegen derartiger Verbände erfordert jedoch fachmännisches Können und Erfahrung und kann deshalb in aller Regel nicht von Laien ohne Taping-Erfahrung ausgeführt werden.

Speziell im Kniegelenkbereich kommt erschwerend hinzu, daß die Kniekehle in den meisten Fällen mit Verbandmaterial bedeckt ist und somit eine Beugung des Knies nicht ausreichend möglich ist. Sogenannte Fertigbandagen aus elastischem Gewebe oder Neoprenen haben den Nachteil, daß diese nicht ausreichend auf der Haut fixiert sind und somit nicht selektiv, sondern nur im großflächigen Areal global wirken können. Eine gezielte Stabilisierung und Führung ist in den meisten Fällen nicht möglich.

US-A-5139476 offenbart eine Bandage zur Stabilisierung der Patella, die mittels zwei Streifen um das Knie herum durch Velcro® Streifen fixierbar ist.

Aufgabe der Erfindung war es deshalb, eine Fertigbandage zur Verfügung zu stellen, die aufgrund ihrer Ausgestaltung, ihres Materials und ihrer Eigenschaffen zur prophylaktischen Stützung und zur selektiven Führung der Patella geeignet ist und die auch vom Verwender in einfacher Weise angelegt werden kann.

Gelöst wird diese Aufgabe durch eine Fertigbandage gemäß Anspruch 1. Die Unteransprüche weisen weitere bevorzugte Ausführungsformen der Fertigbandage gemäß der Erfindung auf.

Als besonders vorteilhaft hat sich eine mittig in dem Streifen angeordnete, kreisförmige Ausnehmung erwiesen, die mit einem der Größe der Patella angepaßten Durchmesser von ungefähr 4 cm versehen ist. Dadurch kann die erfindungsgemäße Fertigbandage universell zur Führung der Patella gegen Lateralisierung, d.h., einem seitlichen Ausweichen der Patella, oder Medialisierung, d.h., einem Ausweichen der Patella parallel zur vertikalen Körperachse, eingesetzt werden.

Der Streifen der Fertigbandage weist eine Länge von circa 1 m auf, vorzugsweise etwa 80 cm, wobei der ungeschlitzte Teil des Streifens etwa 8 cm lang und 10 cm breit ist und die durch den Einschnitt entstandenen beiden Streifen je etwa 68 cm lang und 5 cm breit sind. Mit der Ausnehmung, die eine Länge von circa 4 cm aufweist, ergibt sich somit eine Gesamtlänge der Fertigbandage von etwa 80 cm.

Die Fertigbandage besteht vorzugsweise aus einem längselatischen Gewebe oder Gewirke, das gegebenenfalls auch eine geringe Querelastizität aufweisen kann, insbesondere auf Baumwollbasis. Die Längselastizität entspricht vorzugsweise derjenigen von sogenannten Kurzzugbinden, d.h., Binden mit einer Dehnungsfähigkeit von ungefähr 60-90%.

Um die Beinmuskulatur zu entlasten, sollte das Anlegen der Fertigbandage bei leicht gebeugtem Knie erfolgen. Deshalb weist die ungeschlitzte Schmalseite des Streifens vorzugsweise eine konkave Abrundung auf, die die Beugung des Knies nachvollzieht und somit das Anlegen der Fertigbandage am Kniegelenk erleichtert.

Um ein Abziehen der Fertigbandage vom Kniegelenk zu erleichtern, ist es vorteilhaft, wenn die Enden der beiden durch den Einschnitt getrennten Streifen eine konvexe Abrundung aufweisen.

Die Fertigbandage ist auf der Seite, die auf die Haut aufgelegt wird, mit einer der bekannten gut haftenden Selbstklebemassen auf Basis von Kautschuk oder synthetischen Polymeren beschichtet. Vorteilhaft weisen die Massen weitere Eigenschaften wie gute Hautverträglichkeit oder Luft- und Wasserdampfdurchlässigkeit auf.

Die Klebeschicht ist bis zum Gebrauch der Bandage mit einem klebstoffabweisend ausgerüstetem Blattmaterial wie beispielsweise silikonisiertem Papier oder Folie aus Kunststoff abgedeckt.

Als besonders anwenderfreundlich zeigt sich dabei die Aufteilung der Abdeckung in mehrere einzelne Teile, vorzugsweise dreiteilig. Ein Teil deckt den nicht geschlitzten Teil ab und je ein weiteres streifenförmiges Teil die schmalen Streifen der Fertigbandage. Um die Applikation weiter zu erleichtern, können diese einzelnen Abdeckteile noch entsprechend optisch hervorgehoben sein.

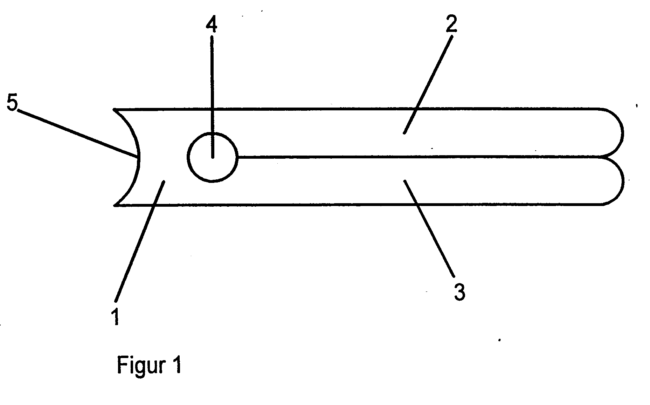

Die Figur 1 zeigt die Fertigbandage in ihrer bevorzugten Ausführungsform. Die Fertigbandage setzt sich aus dem ungeschlitzten Teil (1) sowie den durch den Einschnitt entstandenen Streifen (2) und (3), den sogenannten Zügeln, zusammen. Der ungeschlitzte Teil (1) weist eine konkave Abrundung (5) auf, die beiden Streifen (2) und (3) eine konvexe.

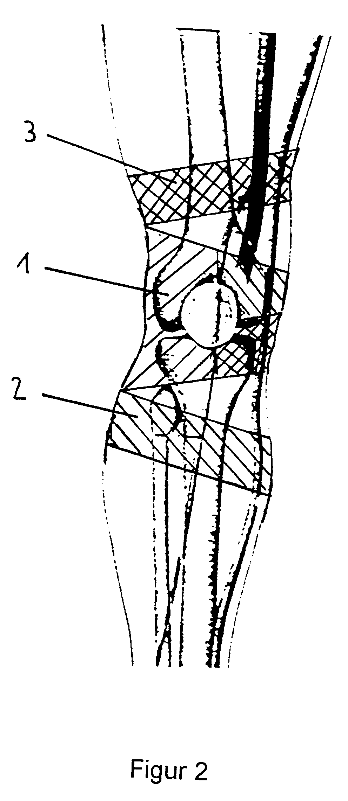

In der Figur 2 wird die bevorzugte Ausführungsform der Fertigbandage dargestellt, wie sie am Kniegelenk zur Fixierung der Patella gegen Lateralisierung angelegt wird. Im ersten Schritt wird das Abdeckpapier von dem ungeschlitzten Teil (1) der Fertigbandage abgezogen. Der ungeschlitzte Teil (1) der Fertigbandage wird anschließend lateral derartig an dem in einem leichten Winkel von vorzugsweise 20°-30° gebeugten Kniegelenk angelegt, daß der kreisförmige Ausschnitt (4) in der Fertigbandage die Patella fest umschließt und die beiden Zügel (2) und (3) in mediale Richtung zeigen.
Nach Entfernung des Abdeckpapiers am Zügel (2), der an der Patella proximal/ventral anliegt, wird dieser nach distal in einer leichten Spiralbewegung um das Bein geführt. Die dorsale Führung des Zügels (2) ist dabei so gelegt, daß die Kniekehle weitgehend freibleibt. Das Ende des Zügels (2) am Unterschenkel befindet sich auf der ventralen Seite.
Analog erfolgt das Anlegen des distal/ventral an der Patella anliegenden Zügels (3), nachdem vorher das Abdeckpapier abgezogen worden ist. Der Zügel (3) wird nach proximal in einer Spiralbewegung am Bein angelegt. Am medialen Teil der Patella überkreuzen sich die beiden Zügel (2) und (3), um somit der Patella den notwendigen Hart zu geben. Auch der zweite Zügel (3) ist derartig gelegt, daß zum einen der dorsale Anteil die Kniekehle weitgehend freiläßt und zum anderen sich das Ende desselben auf der ventralen Seite am Oberschenkel befindet.

Durch die beschriebene Art der Applikation der Fertigbandage wird die Patella stabilisiert und gleichzeitig einer Lateralisierung dieser entgegengewirkt. Der Zügel (2) hält distal lateral gegen die Patella, der Zügel (3) proximal lateral. Damit wird eine ungewollte laterale Bewegung der Patella weitgehend ausgeschlossen.

Alternativ kann die Fertigbandage aber auch eine Medialisierung der Patella einschränken, indem der ungeschlitzte Teil (1) der Fertigbandage distal oder proximal an der Patella fest angelegt wird und die Zügel (2) und (3) anschließend analog zu dem oben Beschriebenen verklebt werden.

## Patentansprüche

1. Fertigbandage zur Stabilisierung und Führung der Patella, die aus einem länglichen Streifen besteht, der annähernd über die gesamte Länge einseitig in Längsrichtung einen Einschnitt aufweist und die eine bei am Knie angelegter Fertigbandage zur Aufnahme der Patella dienende Ausnehmung (4) aufweist, dadurch gekennzeichnet, daß, sich am inneren Ende des Einschnitts die Ausnehmung (4) befindet, und daß, die Fertigbandage auf einer Seite eine selbstklebende Schicht aufweist.

2. Selbstklebende Fertigbandage gemäß Anspruch 1, dadurch gekennzeichnet, daß die Ausnehmung (4) die Form eines Kreises mit einen Durchmesser von ungefähr 4 cm aufweist, der bezogen auf die Breite des Streifens mittig in diesem angeordnet ist.

3. Selbstklebende Fertigbandage gemäß Anspruch 1, dadurch gekennzeichnet, daß der ungeschlitzte Teil (1) des Streifens etwa 8 cm lang und 10 cm breit ist und die durch den Einschnitt entstandenen beiden Streifen (2) und (3) je etwa 68 cm lang und 5 cm breit sind.

4. Selbstklebende Fertigbandage gemäß einem der Ansprüche 1 bis 3, dadurch gekennzeichnet, daß die Fertigbandage universell zur Führung der Patella gegen Lateralisierung, d.h., einem seitlichen Ausweichen der Patella, oder Medialisierung, d.h., einem Ausweichen der Patella parallel zur vertikalen Körperachse, einsetzbar ist.

5. Selbstklebende Fertigbandage gemäß einem der Ansprüche 1 bis 4, dadurch gekennzeichnet, daß die Fertigbandage aus einem längselastischen Gewebe oder Gewirke besteht.

6. Selbstklebende Fertigbandage gemäß einem der Ansprüche 1 bis 5, dadurch gekennzeichnet, daß der ungeschlitzte Teil (1) des Streifens eine konkave Abrundung (5) aufweist.

7. Selbstklebende Fertigbandage gemäß einem der Ansprüche 1 bis 6, dadurch gekennzeichnet, daß die Enden der beiden durch den Einschnitt getrennten Streifen (2) und (3) eine konvexe Abrundung aufweisen.

8. Selbstklebende Fertigbandage gemäß einem der Ansprüche 1 bis 7, dadurch gekennzeichnet, daß die Fertigbandage auf ihrer selbstklebenden Seite mit klebstoffabweisendem Material abgedeckt ist.

9. Selbstklebende Fertigbandage gemäß Anspruch 8, dadurch gekennzeichnet, daß das klebstoffabweisende Material dreiteilig ausgebildet ist, wobei ein Teil den nicht geschlitzten Teil abdeckt und je ein weiteres Teil die schmalen Streifen (2, 3).

## Claims

1. Ready-to-use support tor stabilizing and guiding the patella, which consists of an elongate strip which has, approximately over the entire length, a slit in the longitudinal direction on one side and a cutout (4) serving to receive the patella when the ready-to-use support is applied to the knee, characterized in that the cutout (4) is located at the inner end of the slit and in that the ready-to-use support has a self-adhesive layer on one side.

2. Self-adhesive ready-to-use support according to Claim 1, characterized in that the cutout (4) has the shape of a circle with a diameter of approximately 4 cm which, relative to the width of the strip, is located in the middle of the latter.

3. Self-adhesive ready-to-use support according to Claim 1, characterized in that the unslit part (1) of the strip is about 8 cm long and 10 cm wide, and the two strips (2) and (3) produced by the slit are each about 68 cm long and 5 cm wide.

4. Self-adhesive ready-to-use support according to any of Claims 1 to 3, characterized in that the ready-to-use support can be employed universally for guiding the patella to prevent lateralization, that is to say displacement sideways of the patella, or medialization, that is to say displacement of the patella parallel to the vertical axis of the body.

5. Self-adhesive ready-to-use support according to any of Claims 1 to 4, characterized in that the ready-to-use support consists of a longitudinally elastic woven or knitted fabric.

6. Self-adhesive ready-to-use support according to any of Claims 1 to 5, characterized in that the unslit part (1) of the strip has a concave curvature (5).

7. Self-adhesive ready-to-use support according to any of Claims 1 to 6, characterized in that the ends of the two strips (2) and (3) separated by the slit have a convex curvature.

8. Self-adhesive ready-to-use support according to any of Claims 1 to 7, characterized in that the ready-to-use support is covered on its self-adhesive side with abherent material.

9. Self-adhesive ready-to-use support according to Claim 8, characterized in that the abherent material is designed in three parts, one part covering the unslit part and one other part in each case covering the narrow strips (2, 3).

## Revendications

1. Bandage préfabriqué pour stabiliser et guider la rotule, se composant d'une bande longitudinale, qui présente approximativement sur toute la longueur, d'un côté dans le sens longitudinal une découpure, et qui présente un évidement (4) servant à recevoir la rotule lorsque le bandage préfabriqué est appliqué contre le genou, caractérisé en ce que l'évidement (4) se trouve à l'extrémité interne de la découpure, et en ce que le bandage préfabriqué présente d'un côté une couche auto-adhésive.

2. Bandage préfabriqué auto-adhésif selon la revendication 1, caractérisé en ce que l'évidement (4) a la forme d'un cercle d'approximativement 4 cm de diamètre, qui est disposé au milieu par rapport à la largeur de la bande.

3. Bandage préfabriqué auto-adhésif selon la revendication 1, caractérisé en ce que la partie non fendue (1) de la bande mesure environ 8 cm de long et 10 cm de large et les deux bandes (2) et (3) résultant de la découpure mesurent chacune environ 68 cm de long et 5 cm de large.

4. Bandage préfabriqué auto-adhésif selon l'une quelconque des revendications 1 à 3, caractérisé en ce que le bandage préfabriqué peut être utilisé de manière universelle pour guider la rotule à l'encontre de la latéralisation, c'est-à-dire un affaiblissement latéral de la rotule, ou de la médialisation, c'est-à-dire un affaiblissement de la rotule parallèlement à l'axe vertical du corps.

5. Bandage préfabriqué auto-adhésif selon l'une quelconque des revendications 1 à 4, caractérisé en ce que le bandage préfabriqué se compose d'une étoffe tissée ou d'un tissu à mailles à elasticité longitudinale.

6. Bandage préfabriqué auto-adhésif selon l'une quelconque des revendications 1 à 5, caractérisé en ce que la partie non fendue (1) de la bande présente un arrondi concave (5).

7. Bandage préfabriqué auto-adhésif selon l'une quelconque des revendications 1 à 6, caractérisé en ce que les extrémités des deux bandes (2) et (3) séparées par la découpure présentent un arrondi convexe.

8. Bandage préfabriqué selon l'une quelconque des revendications 1 à 7, caractérisé en ce que le bandage préfabriqué est recouvert, sur son côté auto-adhésif, d'un matériau anti-adhésif.

9. Bandage préfabriqué auto-adhésif selon la revendication 8, caractérisé en ce que le matériau anti-adhésif est conçu en trois parties, une partie recouvrant la partie non fendue et une partie recouvrant respectivement chaque bande mince (2, 3).
